# EUROPEAN PATENT APPLICATION

(11) **EP 3 231 319 A1**
(43) Date of publication of application: **18.10.2017**
(21) Application number: 15867120.6
(22) Date of filing: 13.10.2015
(51) Int. Cl.: A45D 34/04

(54) **COSMETIC CONTAINER HAVING ABSORPTION MEMBER IMPREGNATED WITH LIQUID CONTENTS**

(30) Priority: 12.12.2014 KR 20140179098
(71) Applicant: YONWOO CO.,LTD, 13 Gajwa-ro 84beon-gil Seo-gu Incheon 22824 (KR)
(72) Inventor: LEE, Jae Ock, Incheon 21307 (KR)
(74) Representative: Nordic Patent Service A/S
(86) International application number: PCT/KR2015/010769
(87) International publication number: WO 2016/093482

(57) **Abstract**

The present invention disclosed herein relates to a cosmetic container having an absorption member impregnated with liquid contents, characterized in that the absorption member, made of a porous material and impregnated with liquid contents, is compressed when a piston ascends with the rotation of a rotation body, whereby the impregnated liquid contents are withdrawn to an upper portion thereof for being used, thereby providing the same usability as solid lip stick products which are familiar to users and improving spreading properties.

## Description

### BACKGROUND

The present invention disclosed herein relates to a cosmetic container having an absorption member impregnated with liquid contents, more specifically the cosmetic container having an absorption member impregnated with liquid contents wherein the absorption member, made of a porous material and impregnated with liquid contents, is compressed when a piston ascends with the rotation of a rotation body, whereby the impregnated liquid contents are withdrawn to an upper portion thereof for being used, thereby providing the same usability as solid lip stick products which are familiar to users and improving spreading properties.

Generally, cosmetic products such as lipsticks is configured to open a cap, rotate a rotation body provided at a lower portion of a container thereof, and raise/lower a solid lipstick to the outside of the container. For example, when a user wants to use a lipstick, firstly the user opens a cap of a container, rotates a rotation body, and raises the lipstick to the outside to be protruded. After using the lipstick, the user lowers the lipstick, and makes the lipstick received in the container.

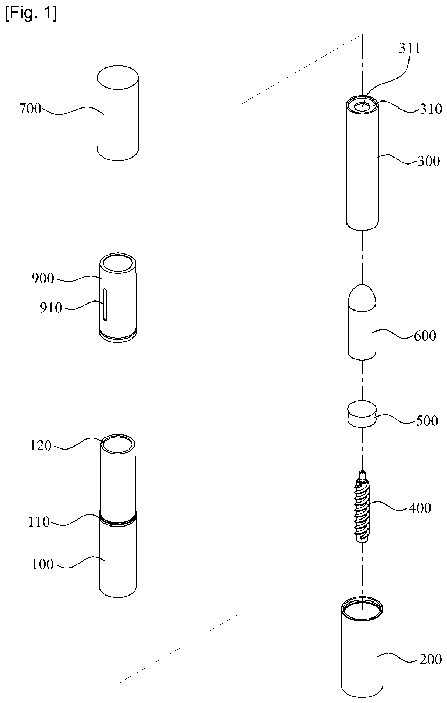

"A lipstick container" with a configuration as the above is disclosed in Fig. 1 of the registered utility model no. 20-0241614 (hereafter called as the registered utility model).

The registered utility model is characterized in that a lipstick (2) is fitted into a lipstick holder (3), and the lipstick holder (3) and a rotation tube (5) are coupled as a fixing protrusion (4) of the lipstick holder (3) is being fitted into a helical groove (6) formed at an inner portion of the rotation tube (5). A guide tube (7) is inserted between the lipstick holder (3) and the rotation tube (5), and coupled to the rotation tube (5) by an annulus protrusion (5). Meanwhile, the fixing protrusion (4) of the lipstick holder (3) is disposed inside a guide groove (8) of the guide tube (7) and, at the same time, at the helical groove (6).

The registered utility model is configured in a way that the fixing protrusion (4) hung between the helical groove (6) and the guide groove (8) ascends when a user holds the guide tube (7) with a hand and rotates the rotation tube (5), and thereby the lipstick (2) fitted in the lipstick holder (3) ascends along for applying lip make up by means of the lipstick (2); however, this type of lipsticks composed of a solid lipstick as in the above has a problem in that ingredients of the lipstick are likely to be spoiled by being contacted with air.

Due to this, recently, there have been developed a variety of lipstick containers which are possible to minimize decomposition of contents thereof by using liquid lipstick with the lipstick smeared on a brush like a manicure or using the liquid lipstick discharged by squeezing the liquid lipstick stored in the container with a piston.

### SUMMARY OF THE INVENTION

The present invention is devised to solve the said problems above, and its goal is to provide a cosmetic container having an absorption member impregnated with liquid contents wherein the absorption member impregnated with liquid contents is compressed when a piston ascends with the rotation of a rotation body, whereby the impregnated liquid contents are withdrawn to an upper portion thereof for being used, thereby providing the same usability as solid lip stick products familiar to users and improving spreading properties.

To solve the above problems, a cosmetic container having an absorption member impregnated with liquid contents according to the present invention includes: an outer container; a rotation body encasing a lower portion of the outer container and rotatably coupled, further provided with an ascending/descending guide member which extends upwards from an inner lower end thereof; an inner container which ascends/descends according to rotation of the rotation body at an inner side of the outer container; a piston rod which is screw-coupled to the ascending/descending guide member and ascends/descends according to rotation of the rotation body; a piston which is coupled to an upper portion of the piston rod and moves along the ascent/descent of the piston rod, and closely contacted to an inner wall of the inner container; an absorption member made of porous material impregnated with liquid contents; and an over cap encasing an upper portion of the outer container and rotatably coupled to the outer container,

characterized in that, when the rotation body is rotated to one side direction, the inner container ascends along while the piston rod and the piston ascend and then an upper portion of the absorption member is firstly exposed to the outside of the outer container, and when the rotation body is further rotated in a state that the upper portion of the absorption member is exposed to the outside, the piston ascends along an inner wall of the inner container in a state that the ascent of the inner container is limited and squeezes the absorption member, such that liquid contents impregnated in the absorption member are withdrawn to an upper portion thereof for being used.

Furthermore, it is characterized in that at an upper portion of the outer container is provided a limiting protrusion which supports an upper end of the inner container when the inner container ascends, and thereby limits the ascent of the inner container.

Furthermore, it is characterized in that at an inner side of the inner container is provided a fixing part which is formed with a hollow for the absorption member being fitted-coupled and fixed, wherein the fixing part prevents the absorption member from moving to an upper portion thereof when the piston ascends and presses the absorption member.

Furthermore, it is characterized in that at an inner upper side of the over cap is provided a sealing cap which seals an opened upper portion of the outer container such that liquid contents impregnated in the absorption member are prevented from being vaporized.

Furthermore, it is characterized to include a decoration part which is coupled encasing an upper portion of the outer container and formed with a transparent window for being able to discern the color of liquid contents impregnated in the absorption member or the ascent/descent of the piston from the outside.

As mentioned in the above, the present disclosure is configured in a way that, when the piston ascends according to rotation of the rotation body, an absorption member made of porous material impregnated with liquid contents is squeezed and thereby the liquid contents impregnated is withdrawn to an upper portion of the absorption member for being used, thereby improving spreading properties and providing the same usability as solid lip stick products familiar to users.

### BRIEF DESCRIPTION OF THE DRAWINGS

Fig. 1 is an exploded perspective view illustrating a configuration of a cosmetic container having an absorption member impregnated with liquid contents according to an exemplary embodiment of the present invention.
Fig. 2 is an assembled perspective view illustrating a configuration of a cosmetic container having an absorption member impregnated with liquid contents according to an exemplary embodiment of the present invention.
Fig. 3 is an assembled cross-sectional view illustrating a configuration of a cosmetic container having an absorption member impregnated with liquid contents according to an exemplary embodiment of the present invention.
Figs. 4 to 6 are explanatory drawings illustrating using methods of a cosmetic container having an absorption member impregnated with liquid contents according to an exemplary embodiment of the present invention.

### DETAILED DESCRIPTION OF PREFERRED EMBODIMENTS

Hereinafter, exemplary embodiments of the present invention will be described in detail with reference to the accompanying drawings. The same reference numerals provided in the drawings indicate the same members.

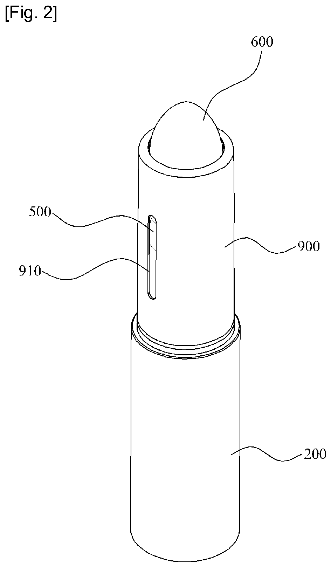

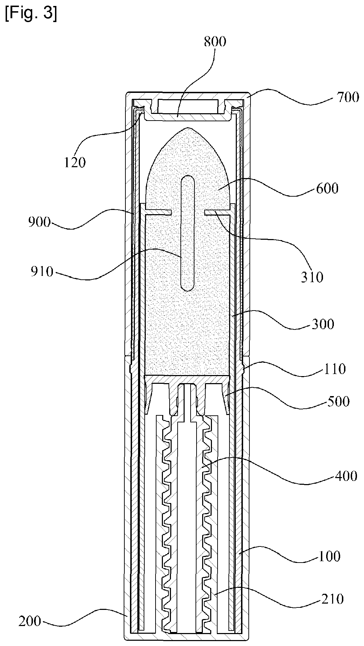

Fig. 1 is an exploded perspective view illustrating a configuration of a cosmetic container having an absorption member impregnated with liquid contents according to an exemplary embodiment of the present invention. Fig. 2 is an assembled perspective view illustrating a configuration of a cosmetic container having an absorption member impregnated with liquid contents according to an exemplary embodiment of the present invention. Fig. 3 is an assembled cross-sectional view illustrating a configuration of a cosmetic container having an absorption member impregnated with liquid contents according to an exemplary embodiment of the present invention.

Referring to Figs. 1 to 3, a cosmetic container having an absorption member impregnated with liquid contents according to an exemplary embodiment of the present invention includes an outer container 100, a rotation body 200, an inner container 300, a piston rod 400, a piston 500, an absorption member 600, and an over cap 700.

The outer container 100, receiving an inner container 300, has a cylindrical shape with a predetermined length such that the inner container 300 can ascend/descend at an interior thereof, further having a coupling protrusion 110 formed at an outer lower portion such that the rotation body 200 can be rotatably coupled therein.

In the present invention, it is characterized in that at an upper portion of the outer container 100 is provided a limiting protrusion 120 which limits the ascent of the inner container 300, wherein the limiting protrusion 120 supports an upper end of the inner container 300 when the inner container 300 ascends by rotation of the rotation body 200, such that it is possible for the piston 500 to press the absorption member 600 as ascending along an inner wall of the inner container 300 when the rotation body continues rotating.

Meanwhile, at an upper portion of the outer container 100 is provided a decoration part 900 which has a transparent window 910 for discerning from the outside the color of liquid contents impregnated in the absorption member 600 or the ascent/descent of the piston 500. At this time, it is preferable that the outer container 100 should be made of a transparent material.

The rotation member 200 is rotatably coupled, encasing a lower portion of the outer container 100, and, in the present invention, is characterized to be provided with an ascending/descending guide member 210, at an inner side of the rotation body 200, which extends upwards from a lower end thereof.

The ascending/descending guide member 210 is provided with a screw thread at an inner circumferential surface thereof and is screw-coupled with the piston rod 400, which will make it possible for the piston rod 400 to ascend/descend according to rotation directions of the rotation body 200.

The inner container 300, ascending/descending at an inner side of the outer container 100 according to rotation of the rotation body 200, moves along together with the ascent/descent of the piston 500 which is formed in a close contact to an inner wall of the inner container; when ascending, the inner container 300 is supported by the limiting protrusion 120 and limited in ascending, and when descending, the inner container 300 is supported by a bottom surface of the rotation body 200 and limited in descending.

The present invention is characterized in that at an inner side of the inner container 300 is provided a fixing part 310 which forms a hollow 311 such that the absorption member 600 can be fitted-coupled and fixed, wherein the fixing part 310 prevents the absorption member 600 from moving to an upper portion thereof when the piston 500 ascends and presses the absorption member 600.

The inner container 300 is preferably made of a transparent material such that it is possible to discern from the outside the color of liquid contents impregnated in the absorption member 600 or the ascent/descent of the piston 500.

The piston rod 400 is screw-coupled to the ascending/descending guide member 210 and ascends/descends according to rotation of the rotation body 200, further including a screw-thread at an outer circumferential surface for being capable of screw-coupling with the ascending/descending guide member 210.

The piston 500, coupled to an upper portion of the piston rod 400 and moving along together with the ascent/descent of the piston rod 400, is configured to ascend/descend in a close contact with an inner wall of the inner container 300. Due to this, when ascending, the piston 500 pressurizes the absorption member 600 which is disposed at an upper portion thereof, such that liquid contents impregnated in the absorption member 600 can move to an upper portion of the absorption member 600.

Furthermore, the piston 500 is closely contacted to an inner wall of the inner container 300, such that, when the piston 500 pressurizes the absorption member 600, the liquid contents impregnated in the absorption member 600 is prevented from moving to a lower portion along an inner wall of the inner container 300.

The absorption member 600 is fitted-coupled with a fixing part 310 inside of the inner container 300. In the present invention, the absorption member 600 is characterized to be composed of a porous material impregnated with liquid contents.

The absorption member 600 is made of an elastic material which gets contracted by compression generated by the ascent of the piston 500 and then is released when the piston 500 descends. It is preferred that an upper portion of the absorption member 600 should be configured to have a shape which gets narrower towards an upper portion thereof in order that a user can easily apply liquid contents onto her lips.

The over cap 700, which is detachably coupled to a decoration part 900 as encasing an upper portion of the outer container 100, is coupled to a sealing cap 800, at an upper inner side thereof, which seals an opened upper portion of the outer container 100 so as to prevent liquid contents impregnated in the absorption member 210 from being vaporized. The over cap 700, in case there is no decoration part 900, can be detachably coupled to the outer container 100.

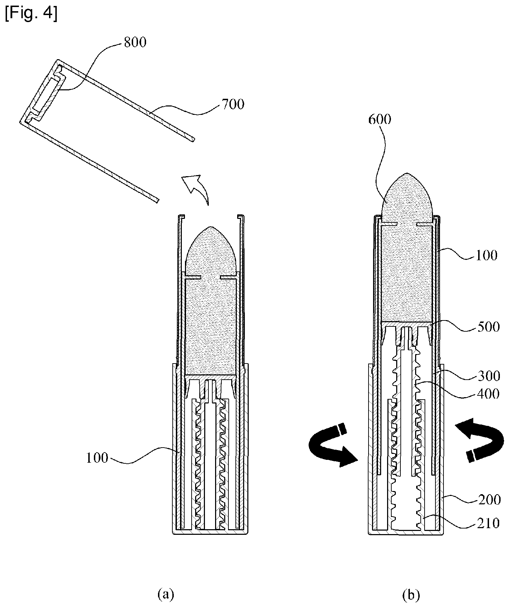

Hereafter, referring Figs. 4 to 6, a using method of to a cosmetic container having an absorption member impregnated with liquid contents according to an exemplary embodiment of the present invention will be explained. Figs. 4 to 6 are explanatory drawings illustrating using methods of a cosmetic container having an absorption member impregnated with liquid contents according to an exemplary embodiment of the present invention.

Firstly, referring Fig. 4, when a user pressurizes a decoration part 900 with one hand in a state of detaching the over cap 700 from the decoration part 900 and rotates the rotation body 200 to one side direction with the other hand, there arise an ascent of the piston rod 400 which is screw-coupled with the ascending/descending guide member 210. When the piston rod 400 ascends, the piston 500 coupled to an upper portion of the piston rod 400 ascends and thereby the inner container 300 ascend along together according to the piston 500 ascending.

As in the above, when the inner container 300 ascends, an absorption member 600 fixed to a fixing part 310 of the inner container 300 ascends along together; due to this, an upper portion of the absorption member 600 is withdrawn and gets exposed to the outside of the outer container 100.

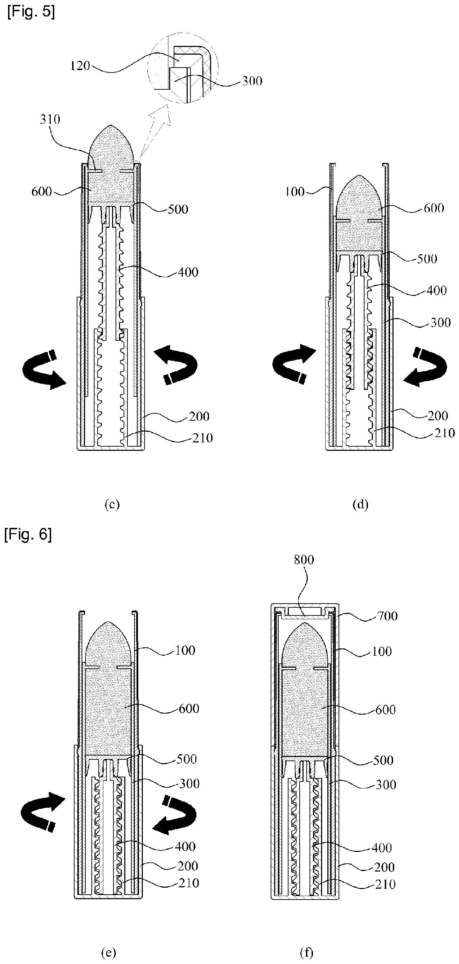

Next, when the rotation body 200 is rotated further in a state that the absorption member 600 is exposed to the outside, as illustrated in Fig. 5(c), the piston 500 ascends along an inner wall of the inner container 300 and pressurizes the absorption member 600, in a state that an upper portion of the inner container 300 is supported by a limiting protrusion 120 of the outer container 100 and thereby the ascent of the inner container 300 is limited, and thereby moves liquid contents impregnated in the absorption member 600 to the upper portion thereof. Due to this, it is possible to apply liquid contents having moved to the upper portion of the absorption member 600 onto lips.

Next, as illustrated in Fig. (d), when the rotation body 200 is rotated to the other direction, the piston rod 400 screw-coupled to the ascending/descending guide member 210 descends. When the piston rod 400 gets lowered, the piston 500 coupled to an upper portion thereof descends and thereby the inner container 300 descends along together as the piston 500 is descending.

As in the above, when the inner container 300 descends, the absorption member 600 fixed to the fixing part 310 of the inner container 300 descends along together; due to this, the absorption member 600 is inserted into an interior of the outer container 100.

Next, the absorption member 600 rotates the rotation body 200 further in a state the absorption member 600 is inserted into the outer container 100, as illustrated in Fig. 6(e), the piston 500 descends along the inner wall of the inner container 300 in a state that a lower portion of the inner container 300 is supported by a bottom surface of the outer container 100 and the descent of the inner container 300 is limited; due to this, the pressure that pressurizes the absorption member 600 is released and thereby the absorption member 600 is relieved and restored.

As in the above, the absorption member 600, when restored in a state being inserted into the outer container 100, is coupled with the over cap 700 and seals an opened upper portion of the outer container 100 through a sealing cap 800 provided at an inner side thereof, therefore preventing liquid contents impregnated in the absorption member 600 from being vaporized.

As previously described, since the absorption member 600 of the present invention is impregnated with liquid contents, basically it is possible to use liquid contents impregnated in an upper portion of the absorption member 600. When liquid contents impregnated in the upper portion of the absorption member 600 are lacked, it is possible to withdraw liquid contents to the upper portion of the absorption member 600 through compression of the absorption member 600 by the ascent of the piston 500 and to always apply the contents in a state of moisture, such that it is possible to provide excellent spreading properties, and therefore is possible to be fit for a variety of cosmetic goods such as lipsticks, and concealer, etc.

As described above, optimal embodiments have been disclosed in the drawings and the specification. Although specific terms have been used herein, these are only intended to describe the present invention and are not intended to limit the meanings of the terms or to restrict the scope of the present invention as disclosed in the accompanying claims. Accordingly, those skilled in the art will appreciate that various modifications and other equivalent embodiments are possible from the above embodiments. Therefore, the scope of the present invention should be defined by the technical spirit of the accompanying claims.

## Claims

1. A cosmetic container having an absorption member impregnated with liquid contents, comprising:
an outer container (100);
a rotation body (200) rotatably coupled, encasing a lower portion of the outer container (100), further comprising an ascending/descending guide member (210) extending upwards from a lower end of an inner side thereof;
an inner container (300) ascending/descending according to rotation of the rotation body (200) at an inner side of the outer container (100);
a piston rod (400) screw-coupled to the ascending/descending guide member (210) and ascending/descending according to rotation of the rotation body (400);
a piston (500) coupled to an upper portion of the piston rod (400) and moving according to ascent/descent of the piston rod (400), and formed in a close contact with an inner wall of the inner container (300);
an absorption member (600) fitted-coupled and made of a porous material impregnated with liquid contents; and
an over cap (700) encasing an upper portion of the outer container (100) and detachably coupled,
**characterized in that** when the rotation body (200) is rotated to one side direction, the inner container (300) ascends along together with the piston rod (400) and the piston (500) ascending, and an upper portion of the absorption member (600) is firstly exposed to the outside of the outer container (100), and that when the rotation body (200) is further rotated in a state of the upper portion of the absorption member (600) being exposed to the outside, the piston (400) ascends along an inner wall of the inner container (300) in a state of an ascent of the inner container (300) being limited and presses the absorption member (600), thereby withdrawing liquid contents impregnated in the absorption member (600) for being used.

2. The cosmetic container having an absorption member impregnated with liquid contents of claim 1, further comprising a limiting protrusion (120) supporting an upper end of the inner container (300) when the inner container ascends and thus limiting ascent of the inner container.

3. The cosmetic container having an absorption member impregnated with liquid contents of claim 1, further comprising a fixing part (310) having a hollow (311) for the absorption member (600) being fitted-coupled and fixed, wherein the fixing part (310) is characterized to prevent the absorption member (600) from moving upwards when the piston (500) ascends and presses the absorption member (600).

4. The cosmetic container having an absorption member impregnated with liquid contents of claim 1, further comprising a sealing cap (800) sealing an opened upper portion of the outer container (100) for preventing liquid contents impregnated in the absorption member (600) from being vaporized.

5. The cosmetic container having an absorption member impregnated with liquid contents of claim 1, further comprising a decoration part (900), coupled encasing an upper portion of the outer container and provided with a transparent window (910) for discerning from the outside a color of liquid contents impregnated in the absorption member (600) or ascent/descent of the piston (500).
